# EUROPEAN PATENT APPLICATION

(11) **EP 4 603 184 A1**
(43) Date of publication of application: **20.08.2025**
(21) Application number: 23876683.6
(22) Date of filing: 10.10.2023
(51) Int. Cl.: B01J 23/888, B01J 23/88, B01J 27/24, B01J 37/08, C07C 31/20, C07C 29/00

(54) **HETEROGENEOUS DUAL-CATALYTIC-CENTER CATALYST, PREPARATION METHOD THEREFOR, AND USE THEREOF**

(30) Priority: 10.10.2022 CN 202211236757
(71) Applicant: China Petroleum & Chemical Corporation, Beijing 100728 (CN); Sinopec (Shangai) Research Institute of Petrochemical Technology Co., Ltd., Shanghai 201208 (CN)
(72) Inventor: YANG, Weimin, Shanghai 201208 (CN); XU, Rui, Shanghai 201208 (CN); LI, Xiangcheng, Shanghai 201208 (CN); WANG, Zhendong, Shanghai 201208 (CN); JIA, Yuqing, Shanghai 201208 (CN)
(74) Representative: karo IP
(86) International application number: PCT/CN2023/123714
(87) International publication number: WO 2024/078481

(57) **Abstract**

The present invention relates to the technical field of catalytic chemistry, and in particular to a catalyst with dual catalytic centers, and a preparation method and use thereof, as well as a method for producing glycols. The catalyst comprises: a porous support and C₃N₄, a hydrogenation metal and a retro-aldol catalytic metal loaded on the porous support; the hydrogenation metal and the retro-aldol catalytic metal form clusters with C₃N₄. The hydrogenation metal and the retro-aldol catalytic metal are loaded on the porous support by coordination with the N site, and form clusters with C₃N₄, effectively stabilizing the metal active sites. The catalyst is used for producing glycols from a polyhydroxy compound. The raw material conversion rate and product glycol selectivity are both very high. The catalyst has high cycling stability, and no deactivation of the catalyst is observed after four cycles of use.

## Description

### TECHNICAL FIELD

The present invention relates to a heterogeneous catalyst with dual catalytic centers, and a preparation method and use thereof, in particular to use in a method for producing glycols.

### BACKGROUND ART

Glycols are common bulk chemicals, among which ethylene glycol is an important basic chemical raw material for producing polyester materials (such as PET, PEF, etc.), antifreeze, and coolants. Currently, the conventional industrial production route for ethylene glycol mainly relies on petroleum or natural gas as raw materials. This route suffers from disadvantages such as dependence on fossil fuels, high energy consumption, and high carbon dioxide emissions. However, these disadvantages can be avoided by using sugar-based biomass as the starting raw material. Sugar-based biomass is widely present in nature, with abundant and easily accessible sources; however, at present, the technology for catalytically converting sugar-based biomass into ethylene glycol is not yet mature. Therefore, the development of new catalysts and new processes can promote the industrialization of this technology, thereby generating significant environmental and economic benefits.

CN105523890A discloses a method for producing glycols from sugar, wherein glucose is directly used as a raw material, which is subjected to hydrolysis and hydrogenation in the presence of tungstate and alloy hydrogenation catalysts to produce glycols. This method requires alloys as co-catalysts, resulting in high metal usage; and additional tungstate needs to be added to the reaction system as co-catalysts, which will be lost with the solvent. CN102190562A, CN101735014A and CN102731258A mainly use WC₂ and Ni nanoparticles as catalytic active components to convert glucose into ethylene glycol, with a maximum glycol yield of 60%. CN103420796A employs a composite catalyst composed of noble metal Ru/C and tungstic acid, achieving an ethylene glycol yield of 52-57%.

In summary, the existing technologies mainly suffer from problems such as low yield of glycol products or poor catalyst cycling stability, which pose significant challenges for practical industrial applications.

### SUMMARY OF INVENTION

The object of the present invention is to overcome the problems of poor cycling stability of sugar-based glycol catalysts in the prior art by providing a heterogeneous catalyst with dual catalytic centers. This catalyst is used for producing glycols from a polyhydroxy compound and has the advantages of low loss rate of active components and excellent cycling stability.

In order to achieve the above object, the present invention provides a heterogeneous catalyst with dual catalytic centers, which comprises a porous support and a heterogeneously distributed ligand, a heterogeneously distributed hydrogenation metal and a heterogeneously distributed retro-aldol catalytic metal loaded on the porous support; the hydrogenation metal and the retro-aldol catalytic metal form clusters with the ligand, wherein the hydrogenation metal and the retro-aldol catalytic metal coordinate with the coordination element of the ligand, and form aggregates having an average particle diameter in the range of 5-100 nm with the ligand.

A typical example of the ligand according to the present invention is C₃N₄.

For example, the heterogeneous catalyst with dual catalytic centers provided by the present invention is a dual catalytic center catalyst, which comprises: a porous support and C₃N₄, a hydrogenation metal and a retro-aldol catalytic metal loaded on the porous support; the hydrogenation metal and the retro-aldol catalytic metal form clusters with C₃N₄.

The present invention also provides a method for preparing the catalyst according to the present invention, characterized in that the method comprises the following steps:
(1) dissolving a retro-aldol catalytic metal source, a hydrogenation metal source, and a ligand precursor in a solvent, followed by loading onto a porous support through contact with the porous support;
(2) calcining the product obtained in step (1) under an inert gas atmosphere.

For example, the method for preparing the catalyst provided by the present invention comprises:
(1) dissolving a retro-aldol catalytic metal source, a hydrogenation metal source and a C₃N₄ precursor in a solvent, and loading the retro-aldol catalytic metal source, the hydrogenation metal source and the C₃N₄ precursor onto a porous support;
(2) calcining the product obtained in step (1) under an inert gas atmosphere.

The present invention also provides use of the heterogeneous catalyst with dual catalytic centers according to the present invention in the production of glycols from a polyhydroxy compound.

The present invention also provides a method for producing glycols, which comprises hydrolyzing and hydrogenating a polyhydroxy compound in the presence of the heterogeneous catalyst with dual catalytic centers according to the present invention and hydrogen to produce glycols; the catalyst comprises the catalyst according to the present invention.

In the prior art, the porous structure of activated carbon is beneficial for the diffusion of biomass macromolecules, but is not conducive to preventing the loss of metal active sites. In the heterogeneous catalyst with dual catalytic centers according to the present invention, the hydrogenation metal and the retro-aldol catalytic metal are coordinated with the coordination sites (e.g., N sites) of the ligand and loaded on the porous support in the form of heterogeneous distribution, and form clusters with the ligands (e.g., C₃N₄), effectively stabilizing the metal active sites.

In the preparation method of the heterogeneous catalyst with dual catalytic centers according to the present invention, a retro-aldol catalytic metal source, a hydrogenation metal source and a ligand (e.g., C₃N₄) precursor are dissolved in a solvent for pre-coordination and then loaded onto a porous support, so that the hydrogenation metal and the retro-aldol catalytic metal are coordinated with the coordination sites (e.g., N sites) of the ligand and loaded on the porous support, and form clusters with the ligand (e.g., C₃N₄).

When the heterogeneous catalyst with dual catalytic centers according to the present invention is used for producing glycols from a polyhydroxy compound, both the conversion rate of the raw material and the selectivity of the product glycols are very high. Moreover, the hydrogenation metal and the retro-aldol catalytic metal form clusters with the ligand (e.g., C₃N₄), and due to the coordination effect between the metals and the coordination sites (e.g., N sites) of the ligand, the loss of the hydrogenation metal and the retro-aldol catalytic metal during the reaction is significantly reduced, effectively stabilizing the metal active sites and enhancing the cycling stability of the catalyst. No deactivation of the catalyst is observed after four cycles of use.

### BRIEF DESCRIPTION OF DRAWINGS

FIG. 1 is a scanning electron microscope (SEM) image of the NiW-C₃N₄/AC catalyst in Example 1;
FIG. 2 is an X-ray photoelectron spectroscopy (XPS) spectrum of the Ni element contained in the catalyst obtained in Example 1;
FIG. 3 is an X-ray photoelectron spectroscopy (XPS) spectrum of the W element contained in the catalyst obtained in Example 1;
FIG. 4 is an XRD pattern of the catalyst obtained in Example 1;
FIG. 5 is a transmission electron microscope HAADF image, EDS image and particle diameter distribution diagram of the catalyst obtained in Example 1; and
FIG. 6 is a statistical diagram of the average particle diameter of clusters of the catalyst obtained in Example 1.

### DETAILED DESCRIPTION OF INVENTION

The endpoints of the ranges and any values disclosed herein are not limited to the precise ranges or values, and these ranges or values should be understood to include values close to these ranges or values. For numerical ranges, two of the endpoint values of each range, one of the endpoint values of each range and one of individual point values, as well as two of the individual point values, can be combined with each other to obtain one or more new numerical ranges, which should be regarded as specifically disclosed herein.

The present invention provides a heterogeneous catalyst with dual catalytic centers, which comprises a porous support and a heterogeneously distributed ligand, a heterogeneously distributed hydrogenation metal and a heterogeneously distributed retro-aldol catalytic metal loaded on the porous support; the hydrogenation metal and the retro-aldol catalytic metal form clusters with the ligand, wherein the hydrogenation metal and the retro-aldol catalytic metal coordinate with the coordination element of the ligand, and form aggregates having an average particle diameter in the range of 5-100 nm with the ligand.

In the present invention, without special mention, the descriptions of amounts, contents, and related ratios thereof are all based on "weight", unless it is obviously inconsistent with the common understanding in the art.

For the purpose of the present invention, the term "dual catalytic centers" refers to a catalyst on which there are catalytic centers with both of two active functions, i.e. hydrogenation and retro-aldol catalysis are achieved by coordinating both of the hydrogenation metal and the retro-aldol catalytic metal with the coordination element of the ligand and loading both on the same porous support.

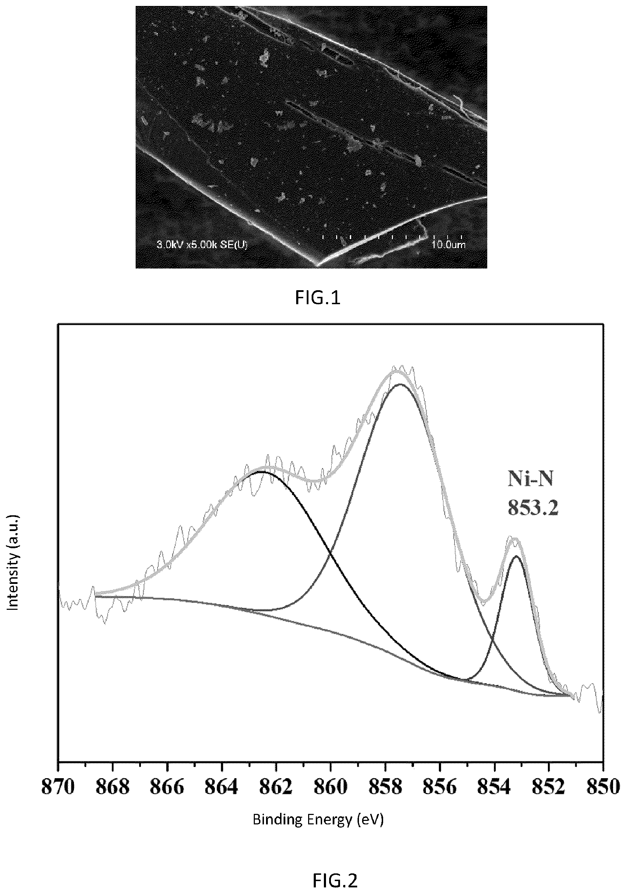

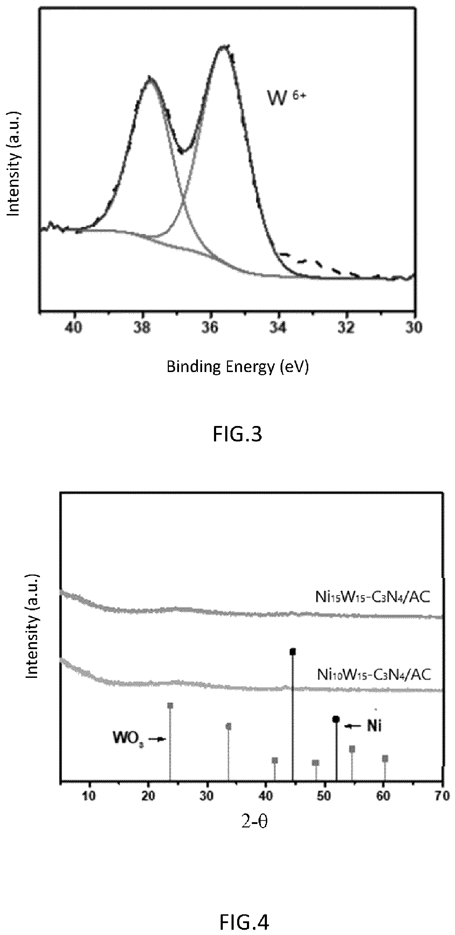

The coordination can be confirmed by those skilled in the art based on the technical knowledge known in the art, for example, from the XPS spectra of the catalyst, as shown in FIG. 2 and FIG. 3.

The term "clusters" has a general meaning known in the art, and for the purpose of the present invention, specifically refers to aggregates having an average particle diameter of 5-100 nm formed from the hydrogenation metal and the retro-aldol catalytic metal with the ligand by the coordination of these metals with the coordination element of the ligand. As an example, a transmission electron microscopy HAADF image can be obtained for the heterogeneous catalyst, and on this basis, the Nano measurer particle diameter analysis software is used to statistically analyze the average particle diameter of the clusters.

For the purpose of the present invention, hydrogenation reaction and retro-aldol reaction each have the meaning known in the art. For example, it is generally known that aldol condensation reaction is a reversible reaction, wherein the forward reaction involves the formation of a hydroxy aldehyde or ketone from an aldehyde or ketone containing active hydrogen under the catalysis of a base or an acid, while the reverse reaction involves the hydrolysis of a hydroxy aldehyde or ketone under the action of a catalyst, i.e., the retro-aldol catalytic reaction.

**In** the art, catalysts used for hydrogenation reactions are generally heterogeneous; however, catalysts suitable for retro-aldol reactions are generally homogeneous. It has been reported that a homogeneous tungsten-containing material is used as a retro-aldol catalyst, for example, see US20110312487. For the purpose of the present invention, "homogeneous" and "heterogeneous" each have the meaning known in the art. A catalyst uniformly dispersed in a reaction medium in the form of dissolution or others, such as a solution, can be regarded as an example of a homogeneous catalyst; a catalyst loaded on particles by coating or others can be regarded as an example of a heterogeneous catalyst.

The inventors of the present invention have unexpectedly found through in-depth research that for the purpose of the present invention, the catalyst with both the hydrogenation and retro-aldol catalysts provided in a heterogeneous form can be prepared by simple steps, especially by achieving simultaneous loading of the hydrogenation metal and the retro-aldol catalytic metal, thereby facilitating the hydrogenation metal and the retro-aldol catalytic metal to play the role of their dual catalytic centers; and this achieves desirable technical effects, such as reactant conversion rate and product yield.

According to the present invention, the ligand may be C₃N₄ or a derivative thereof, wherein the C₃N₄ derivative includes heteroatom (preferably selected from phosphorus and sulfur) doped carbon nitrides, defective carbon nitrides, carbon nitride heterojunctions or carbon nitride copolymers.

According to the present invention, the hydrogenation metal and the retro-aldol catalytic metal are each coordinated with the coordination element of the ligand. For example, when Ni is used as the hydrogenation metal and C₃N₄ is used as the ligand, one Ni atom is at least coordinated with one N atom in the C₃N₄ framework, thereby anchoring in the C₃N₄ framework to form a Ni₁-N active single-site. Similarly, the retro-aldol metal lacks electrons and has an empty orbital; while the coordination site (such as N) on the ligand has a lone pair of electrons, which can be combined with the empty orbital to form coordination.

According to the present invention, each atom of the hydrogenation metal and the retro-aldol catalytic metal can basically be coordinated with the ligand, for example, the molar ratio of uncoordinated metal atoms to the total amount of metal atoms is less than or equal to 10%, preferably less than or equal to 5%, and more preferably less than or equal to 2%.

In order to achieve full coordination of each atom of the hydrogenation metal and the retro-aldol catalytic metal, the molar amount of the coordination elements in the present invention is preferably greater than or equal to the molar amount of the metal atoms. For example, according to the present invention, the molar ratio of the coordination elements to the metal atoms is greater than or equal to 2: 1, preferably greater than or equal to 4:1, and further preferably greater than or equal to 8: 1.

According to a preferred embodiment of the present invention, the clusters have an average particle diameter in the range of 5-100 nm, more preferably 5-30 nm.

According to a preferred embodiment of the present invention, the hydrogenation metal and the retro-aldol catalytic metal are loaded on the support in the form of active single-site of the metals.

According to a preferred embodiment of the present invention, the porous support has an average particle diameter in the range of 0.01-1 mm, preferably 0.1-0.5 mm.

According to a preferred embodiment of the present invention, the porous support has a specific surface area in the range of 200-2000 m²·g⁻¹.

In the present invention, the weight content of the hydrogenation metal in the catalyst can be selected in a wide range. According to a preferred embodiment of the present invention, the content of the hydrogenation metal is in the range of 1%-50%, preferably 6%-30%, based on the total weight of the catalyst.

In the present invention, the weight content of the retro-aldol catalytic metal in the catalyst can be selected in a wide range. According to a preferred embodiment of the present invention, the content of the retro-aldol catalytic metal is in the range of 0.1%-50%, preferably 10%-30%, based on the total weight of the catalyst.

For the purposes of the present invention, the content of each metal in the catalyst can be calculated as follows: Catalyst metal weight content % = (weight content of the metal in raw material) / (weight of the prepared catalyst) × 100%.

In the present invention, the weight content of the porous support in the catalyst can be selected in a wide range. According to a preferred embodiment of the present invention, the content of the porous support is in the range of 25-96.9%, preferably 40-84%, based on the total weight of the catalyst.

In the present invention, the weight content of the ligand (e.g., C₃N₄) in the catalyst can be selected in a wide range. According to a preferred embodiment of the present invention, the content of the ligand (e.g., C₃N₄) is in the range of 2-50%, preferably 10-30%, based on the total weight of the catalyst.

According to a preferred embodiment of the present invention, the weight ratio of the retro-aldol catalytic metal to the hydrogenation metal is in the range of 0.5-6, preferably 0.8-2.5.

According to the present invention, the porous support has a wide range of optional types. Any conventional porous support in the art can be used in the present invention. According to a preferred embodiment of the present invention, the porous support is selected from carbon-based porous supports, preferably selected from at least one of graphene, graphyne, activated carbon fiber, activated carbon and carbon nanotubes, more preferably activated carbon.

According to the present invention, there is no particular limitation on the type of the hydrogenation metal, and any conventional hydrogenation metal in the art can be used in the present invention. According to a preferred embodiment of the present invention, the hydrogenation metal is selected from at least one of Ni, Fe, Cu, Pt, Pd, Ru and Rh, preferably selected from at least one of Ni, Fe and Cu.

According to the present invention, there is no particular limitation on the type of the retro-aldol catalytic metal, and any conventional retro-aldol catalytic metal in the art can be used in the present invention. According to a preferred embodiment of the present invention, the retro-aldol catalytic metal is selected from at least one of W, Mo and Ce.

In the present invention, there is no particular requirement for the preparation method of the catalyst. For example, the present invention provides a method for preparing the catalyst according to the present invention, the method comprising the following steps:
(1) dissolving a retro-aldol catalytic metal source, a hydrogenation metal source, and a ligand (e.g., C₃N₄) precursor in a solvent, followed by loading onto a porous support through contact with the porous support;
(2) calcining the product obtained in step (1) under an inert gas atmosphere.

In the present invention, the retro-aldol catalytic metal source, the hydrogenation metal source and the ligand (e.g., C₃N₄) precursor are dissolved in a solvent for pre-coordination and then loaded onto a porous support, so that the hydrogenation metal and the retro-aldol catalytic metal are coordinated with the coordination sites (e.g., N sites) and loaded on the porous support, and form clusters with the ligand (e.g., C₃N₄).

According to a preferred embodiment of the present invention, step (1) comprises:
i) dissolving the retro-aldol catalytic metal source and the ligand (e.g., C₃N₄) precursor in a first solvent to obtain a first solution; dissolving the hydrogenation metal source and the ligand (e.g., C₃N₄) precursor in a second solvent to obtain a second solution;
ii) impregnating the porous support with the first solution and the second solution, respectively, followed by drying.

According to a preferred embodiment of the present invention, the impregnation process is carried out by equal volume impregnation(s). According to the preparation method of the present invention, a simple impregnation is used, and the obtained catalyst does not need to be reduced with hydrogen, and can be directly calcined and used as a catalyst. The method of the present invention is simple, relies on cheap raw materials, and is suitable for large-scale industrial production.

According to a preferred embodiment of the present invention, the impregnation process is carried out by multiple equal-volume impregnations, and drying is performed after each impregnation. The number of impregnations is adjusted according to the concentration of the impregnation solution and the impregnation target.

According to a preferred embodiment of the present invention, the drying conditions include: a temperature of 50-120°C and a time of 5-15 hours.

There is no special requirement on the number of impregnations, which is determined according to actual needs.

In the present invention, the retro-aldol catalytic metal source, the hydrogenation metal source and the ligand (e.g., C₃N₄) precursor are dissolved in a solvent for pre-coordination. The dissolution rate can also be accelerated by ultrasound, stirring and the like.

In the present invention, there is no particular limitation on the calcination conditions, and any conventional calcination method in the art can be used in the present invention. According to a preferred embodiment of the present invention, the calcination conditions include: a calcination temperature of 300-600°C and a calcination time of 0.5-12 hours.

In the present invention, the weight ratio of the hydrogenation metal source to the ligand (e.g., C₃N₄) precursor can be selected in a wide range. According to a preferred embodiment of the present invention, the weight ratio of the hydrogenation metal source to the ligand (e.g., C₃N₄) precursor is in the range of (0.5-10):1, preferably (1-3):1.

In the present invention, the weight ratio of the retro-aldol catalytic metal source to the ligand (e.g., C₃N₄) precursor can be selected in a wide range. According to a preferred embodiment of the present invention, the weight ratio of the retro-aldol catalytic metal source to the ligand (e.g., C₃N₄) precursor is in the range of (0.5-10):1, preferably (1-3):1.

According to a preferred embodiment of the present invention, the product obtained in step (1) is pretreated before calcination, and the pretreatment conditions include: under an inert gas or air atmosphere, treating at 30-100°C for 0.25-24 hours, preferably treating at 35-90°C for 0.5-18 hours, and more preferably treating at 50-85°C for 6-12 hours.

In the present invention, there is no particular limitation on the type of the ligand (e.g., C₃N₄) precursor, and any conventional ligand (e.g., C₃N₄) precursor in the art can be used in the present invention. According to a preferred embodiment of the present invention, the ligand (e.g., C₃N₄) precursor is selected from at least one of urea, dicyandiamide, melamine, and guanidine hydrochloride, preferably selected from at least two of urea, dicyandiamide, melamine, and guanidine hydrochloride.

According to a preferred embodiment of the present invention, the ligand (e.g., C₃N₄) precursor is selected from mixtures of melamine and guanidine hydrochloride, and more preferably the weight ratio of melamine to guanidine hydrochloride is in the range of 0.5-2:1, for example, 0.5:1, 0.6:1, 0.7:1, 0.8:1, 0.9:1, 1:1, 1.2:1, 1.5:1, 1.8:1, 2:1.

In the present invention, there is no particular limitation on the type of hydrogenation metal source, and any conventional hydrogenation metal source in the art can be used in the present invention. According to a preferred embodiment of the present invention, the hydrogenation metal source is selected from soluble salts of the hydrogenation metal, preferably selected from hydrogenation metal acetates, hydrogenation metal nitrates, hydrogenation metal chlorides, and hydrogenation metal sulfates.

According to a preferred embodiment of the present invention, the hydrogenation metal source is at least one of nickel acetate, nickel nitrate, nickel chloride and nickel sulfate.

In the present invention, there is no particular limitation on the type of the retro-aldol catalytic metal source, and any conventional retro-aldol catalytic metal source in the art can be used in the present invention. According to a preferred embodiment of the present invention, the retro-aldol catalytic metal source is selected from at least one of soluble salts of the retro-aldol catalytic metal, soluble acids of the retro-aldol catalytic metal, and soluble oxides of the retro-aldol catalytic metal.

It is to be understood that, in the present invention, "soluble" means soluble in the respective corresponding solvent.

According to a preferred embodiment of the present invention, the retro-aldol catalytic metal source is selected from at least one of tungstic acid, ammonium metatungstate and tungsten oxide.

In the present invention, there is no particular limitation on the types of various solvents, and any conventional solvents in the art can be used in the present invention. For example, the solvents according to the present invention can be independently selected from at least one of water, methanol, ethanol and ethyl acetate, preferably water and/or methanol.

According to a preferred embodiment of the present invention, the first solvent is methanol and the second solvent is water.

The present invention also provides use of the catalyst according to the present invention in the production of glycols from a polyhydroxy compound. The catalyst according to the present invention is particularly suitable for the production of glycols from a polyhydroxy compound; the polyhydroxy compound is selected from at least one of starch, hemicellulose, sucrose, glucose, fructose, furfural and fructan. When the catalyst according to the present invention is used for the production of glycols from a polyhydroxy compound, both the conversion rate of the raw material and the selectivity of the product glycols are very high, and the catalyst has high cycling stability.

The present invention also provides a method for producing glycols, wherein a polyhydroxy compound is hydrolyzed and hydrogenated in the presence of the catalyst according to the present invention and hydrogen to produce glycols.

According to a preferred embodiment of the present invention, the weight ratio of the polyhydroxy compound to the catalyst according to the present invention is in the range of 10-0.1, preferably 5-0.5.

According to a preferred embodiment of the present invention, the weight ratio of water to the polyhydroxy compound is in the range of 30-300:1.

According to a preferred embodiment of the present invention, the initial filling hydrogen pressure is in the range of 0.5-10 MPa, preferably 1-6 MPa.

According to a preferred embodiment of the present invention, the hydrolysis and hydrogenation conditions include: a reaction temperature of 150-300°C, preferably 200-245°C; a reaction time of 0.5-12 h, preferably 0.5-4 h.

### Examples

In order to facilitate the understanding of the present invention, the present invention lists the following examples, but the examples are only used to facilitate understanding of the present invention and should not be regarded as specific limitations to the present invention.

In the present invention, the reaction product glycols (such as ethylene glycol, propylene glycol, butylene glycol) were qualitatively analyzed by gas chromatography-mass spectrometry (GC-MS), and the yield of the product glycols (such as ethylene glycol, propylene glycol, butylene glycol) and the conversion rate of the reaction raw material glucose were analyzed by gas chromatography (GC). The gas chromatography-mass spectrometer was Agilent 7890A from Agilent Corporation, United States, and the chromatographic column was HP-5 non-polar capillary column (30m, 0.53mm); the gas chromatograph was Agilent 7890B, the detector was hydrogen flame ionization detector (FID), and the chromatographic column was SE-54 capillary column (30m, 0.53mm).

In the present invention, the valence state of the metal loaded on the catalyst was characterized by X-ray photoelectron spectroscopy.

In the present invention, the single-site state of the metals was characterized by spherical aberration corrected electron microscope.

In the present invention, the amount of metal lost after the reaction was characterized by inductively coupled plasma emission spectroscopy (ICP).

The catalyst metal loss rate was calculated as follows: Catalyst metal loss % = (metal content in the solution after reaction) / (metal content in the catalyst in the initial reaction) × 100%.

The conversion rate of glucose was calculated as follows: Glucose conversion rate % = (molar amount of glucose participating in the reaction) / (molar amount of glucose as the initial reaction raw material) × 100%.

The yield of product glycols (ethylene glycol, propylene glycol, butylene glycol) was calculated as follows: Yield of product glycols (%) = (carbon number corresponding to the molar amount of glycols produced by the reaction) / (carbon number corresponding to the molar amount of glucose as the initial reaction raw material) × 100%. Selectivity of product glycols (%) = (carbon number corresponding to the molar amount of glycols produced by the reaction) / (molar amount of glucose participating in the reaction) × 100%.

Testing method for average particle diameter of clusters: Based on the HAADF image obtained by electron microscopy, Nano measurer particle diameter analysis software was used to statistically analyze the average particle diameter of the clusters.

In the following examples, the activated carbon had an average particle diameter of 0.25-1 mm, and a specific surface area of 1682 m²•g⁻¹.

### Example 1

(1) 0.102 g of ammonium metatungstate and 0.1 g of dicyandiamide were dissolved in 3 mL of deionized water under heating, and ultrasonicated for half an hour to prepare 1 part of solution A. 0.107 g of nickel acetate tetrahydrate and 0.1 g of dicyandiamide were dissolved in 3 mL of deionized water under heating, and ultrasonicated for half an hour to prepare 1 part of solution B. The impregnation process was carried out by multiple equal volume impregnations. The impregnation steps were as follows: 1 part of solution A was added to 1 g of activated carbon (AC), shaken until mixed evenly, placed in a drying oven at 80°C for 10 h, and this impregnation step was repeated 2 times; 1 part of solution B was added to 1 g of activated carbon (AC), shaken until mixed evenly, and placed in a drying oven at 80°C for 10 h, and this impregnation step was repeated 6 times. A total of 2 parts of solution A (impregnation with solution A 2 times) and 6 parts of solution B (impregnation with solution B 6 times) were added; the obtained product was pretreated at 70°C for 9 hours under a nitrogen atmosphere.
(2) The obtained sample was heated to 550°C under a nitrogen atmosphere and calcined for 2 h to obtain a Ni_{15%}W_{15%}-C₃N₄/AC catalyst.

The SEM electron micrograph image of the catalyst is shown in FIG. 1. The active sites exist in the pores of the activated carbon, and only a few can be observed on the surface;

The XPS spectra of the catalyst are shown in FIG. 2 and FIG. 3, indicating that Ni and W elements are mainly loaded on the activated carbon in the form of ions coordinated with carbon nitride;

The XRD pattern of the catalyst is shown in FIG. 4, indicating that Ni and W elements are not present in the form of nanoparticles on the support;

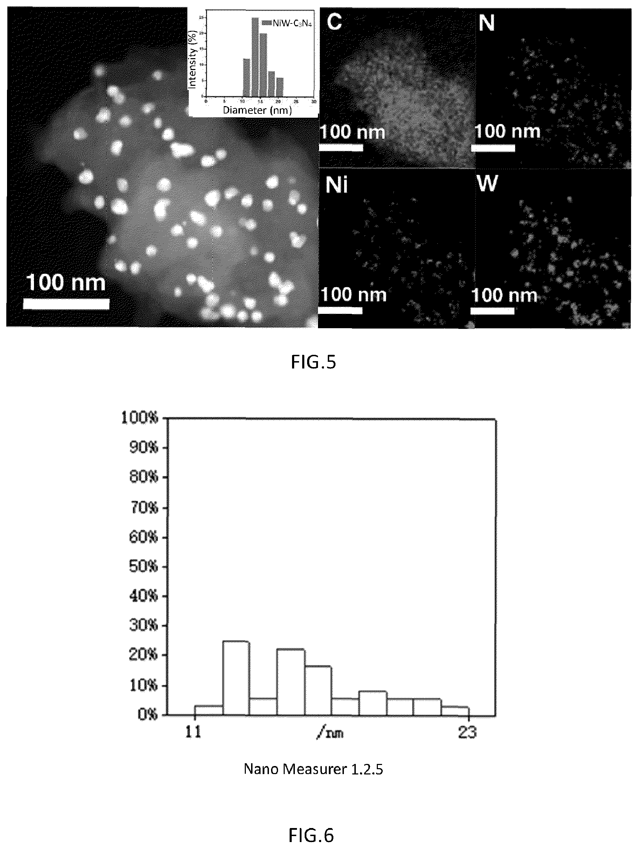

The transmission electron micrograph image of the catalyst is shown in FIG. 5, indicating that the distribution of W, Ni and N sites on the activated carbon in the sample was consistent; the statistical diagram of the average particle diameter of the clusters on the catalyst is shown in FIG. 6, and the average particle diameter of the NiW-C₃N₄ clusters is in the range of 11-23 nm. The catalyst composition and the average particle diameter range of the clusters are shown in Table 1.

### Example 2

Example 2 was conducted following the method of Example 1, except that, in step (1), the impregnation steps were as follows: 1 part of solution A or B was added to 1 g of activated carbon, shaken until mixed evenly, and placed in a drying oven at 80°C for 10h; this impregnation step was repeated 6 times, and a total of 2 parts of solution A and 4 parts of solution B were added; the remaining conditions were the same as those of Example 1, and a Ni_{10%}W_{15%} -C₃N₄/AC catalyst was obtained. The catalyst composition is shown in Table 1.

### Example 3

Example 3 was conducted following the method of Example 2, except that, in step (1), the solvent for dissolving ammonium metatungstate was methanol, specifically: 0.102 g of ammonium metatungstate and 0.1 g of dicyandiamide were dissolved in 3 mL of methanol under heating, ultrasonicated for half an hour to prepare solution A; 0.107 g of nickel acetate tetrahydrate and 0.1 g of dicyandiamide were dissolved in 3 mL of deionized water under heating, ultrasonicated for half an hour to prepare solution B; the remaining conditions were the same as those of Example 2, and a Ni_{10%}W_{15%}-C₃N₄/AC catalyst was obtained. The catalyst composition is shown in Table 1.

### Example 4

Example 4 was conducted following the method of Example 1, except that, in step (1), the impregnation steps were as follows: 1 part of solution A or B was added to 1 g of activated carbon, shaken until mixed evenly, and placed in a drying oven at 80°C for 10h; this impregnation step was repeated 2 times, and a total of 0.33 parts of solution A and 1 part of solution B were added; the remaining conditions were the same as those of Example 1, and a Ni_{2.5%}W_{2.5%}-C₃N₄/AC catalyst was obtained. The catalyst composition is shown in Table 1.

### Example 5

Example 5 was conducted following the method of Example 1, except that, in step (1), methanol was used as the solvent instead of deionized water; the remaining conditions were the same as those of Example 1, and a Ni_{15%}W_{15%}-C₃N₄/AC catalyst was obtained. The catalyst composition is shown in Table 1.

### Example 6

Example 6 was conducted following the method of Example 1, except that, in step (1), ethanol was used as the solvent instead of deionized water; the remaining conditions were the same as those of Example 1, and a Ni_{15%}W_{15%}-C₃N₄/AC catalyst was obtained. The catalyst composition is shown in Table 1.

### Example 7

Example 7 was conducted following the method of Example 1, except that, in step (1), ethyl acetate was used as the solvent instead of deionized water; the remaining conditions were the same as those of Example 1, and a Ni_{15%}W_{15%}-C₃N₄/AC catalyst was obtained. The catalyst composition is shown in Table 1.

### Example 8

Example 8 was conducted following the method of Example 1, except that, in step (1), melamine and guanidine hydrochloride (in a weight ratio of 1:1) were used instead of dicyandiamide with the total amount of C₃N₄ source remaining unchanged to prepare solutions A and B; the remaining conditions were the same as those of Example 1. The catalyst composition is shown in Table 1.

### Example 9

(1) 0.103 g of ammonium molybdate and 0.1 g of dicyandiamide were dissolved in 3 mL of deionized water under heating, and ultrasonicated for half an hour to prepare solution A. 0.135 g of cupric chloride dihydrate and 0.1 g of dicyandiamide were dissolved in 3 mL of deionized water under heating, and ultrasonicated for half an hour to prepare solution B. The impregnation process was carried out by multiple equal volume impregnations. The impregnation steps were as follows: 1 part of solution A or B was added to 1 g of activated carbon (AC), shaken until mixed evenly, and placed in a drying oven at 80°C for 10 h; this impregnation step was repeated 6 times, and a total of 2 parts of solution A and 4 parts of solution B were added;
(2) The obtained sample was heated to 550°C under a nitrogen atmosphere and calcined for 2 h to obtain a Cu_{20%}Mo_{10%}-C₃N₄/AC catalyst. The catalyst composition is shown in Table 1.

### Comparative Example 1

0.102 g of ammonium metatungstate was dissolved in 3 mL of deionized water under heating, and ultrasonicated for half an hour to prepare solution A. 0.107 g of nickel acetate tetrahydrate was dissolved in 3 mL of deionized water under heating, and ultrasonicated for half an hour to prepare solution B. The impregnation process was carried out by multiple equal volume impregnations. The standard steps were as follows: 1 part of solution A or B was added to 1 g of activated carbon, shaken until mixed evenly, and placed in a drying oven at 80°C for 10 h; this step was repeated 6 times during the whole process, and a total of 2 parts of solution A and 6 parts of solution B were added. The obtained sample was heated to 600°C under a nitrogen atmosphere, calcined for 4 h, and then reduced at 450°C under a hydrogen atmosphere for 2 h to obtain a Ni_{15%}W_{15%}/AC catalyst. The catalyst composition is shown in Table 1.

### Comparative Example 2

A Ni_{15%}W_{15%}/AC-C₃N₄ catalyst was prepared by an equal volume impregnation method: 0.102 g of ammonium metatungstate was dissolved in 3 mL of deionized water under heating, and ultrasonicated for half an hour to prepare solution A; 0.107 g of nickel acetate was dissolved in 3 mL of deionized water under heating, and ultrasonicated for half an hour to prepare solution B. The impregnation process was carried out by multiple equal volume impregnations. The standard steps were as follows: 1 part of solution A or B was added to the activated carbon loaded with C₃N₄ (wherein the amount of activated carbon was 1 g and the amount of C₃N₄ was 0.6 g), shaken until mixed evenly, and placed in a drying oven at 80°C for 10 h; this step was repeated 8 times during the whole process, and a total of 2 parts of solution A and 6 parts of solution B were added. The obtained sample was heated to 600°C under a nitrogen atmosphere, calcined for 4 h, and then reduced at 450°C under a hydrogen atmosphere for 2 h to obtain a Ni₁₅W₁₅/AC-C₃N₄ catalyst. The catalyst composition is shown in Table 1.

### Examples 10-18

The reaction of preparing ethylene glycol by catalytic conversion of glucose was carried out in a sealed autoclave. 0.075 g of the catalyst obtained in the above Examples 1-9, 0.25 g of glucose and 25 mL of deionized water were added to an autoclave with a stirrer, hydrogen was introduced to purge the autoclave three times, followed by pressurization with hydrogen to 4 MPa, after which the autoclave was sealed. The heating mantle was raised to 245°C, and then the stirrer was initiated by magnetic stirring. The reaction was carried out at 245°C for 1 h. The products in the reaction solution were quantitatively analyzed by using gas chromatography, and the raw material in the reaction solution was quantitatively analyzed by using liquid chromatography. The conversion rate of glucose and the yield of glycols were calculated according to the above formula, see Table 2. The selectivity of glycols was calculated according to the above formula, see Table 3.

### Comparative Examples 3-4

Comparative Examples 3-4 differ from Examples 10-18 in that the catalyst used in Comparative Example 3 was the Ni_{15%}W_{15%}/AC catalyst prepared in Comparative Example 1; and the catalyst used in Comparative Example 4 was the Ni_{15%}W_{15%}/AC-C₃N₄ catalyst prepared in Comparative Example 2.

**Table 1**

| Catalyst | Ni content (wt %) | W content (wt %) | AC content (wt %) | C₃N₄ content (wt %) | Average particle diameter of NiW-C₃N₄ clusters, nm | Ni valenc e | W valenc e |
|---|---|---|---|---|---|---|---|
| Ex. 1 | 15 | 15 | 43.74 | 26.26 | 18 | +2 | +6 |
| Ex. 2 | 10 | 15 | 45 | 30 | 20 | +2 | +6 |
| Ex. 3 | 10 | 15 | 45 | 30 | 18 | +2 | +6 |
| Ex. 4 | 2.5 | 2.5 | 87 | 8 | 30 | +2 | +6 |
| Ex. 5 | 15 | 15 | 43.74 | 26.26 | 19 | +2 | +6 |
| Ex. 6 | 15 | 15 | 43.74 | 26.26 | 20 | +2 | +6 |
| Ex. 7 | 15 | 15 | 43.74 | 26.26 | 25 | +2 | +6 |
| Ex. 8 | 15 | 15 | 43.74 | 26.26 | 17 | +2 | +6 |
| Ex. 9 | 20 (Cu) | 10 (Mo) | 43.74 | 26.26 | 35 | Cu (+2) | Mo (+6) |
| CE. 1 | 15 | 15 | 70 | 0 | - | 0 | +6 |
| CE. 2 | 15 | 15 | 43.74 | 26.26 | - | 0 | +6 |

**Table 2**

| | Catalyst source | Glucose conversion rate (%) | Glycol yield (%) |
|---|---|---|---|
| Ex. 10 | Ex. 1 | >99 | 80.0 |
| Ex. 11 | Ex. 2 | >99 | 74.8 |
| Ex. 12 | Ex. 3 | >99 | 78.6 |
| Ex. 13 | Ex. 4 | >99 | 61.0 |
| Ex. 14 | Ex. 5 | >99 | 77.3 |
| Ex. 15 | Ex. 6 | >99 | 75.1 |
| Ex. 16 | Ex. 7 | >99 | 70.8 |
| Ex. 17 | Ex. 8 | >99 | 82.2 |
| Ex. 18 | Ex. 9 | >99 | 73.7 |
| CE. 3 | CE. 1 | >99 | 55.3 |
| CE. 4 | CE. 2 | >99 | 56.7 |

**Table 3**

| | Ethylene glycol selectivity (%) | 1,2-Propylene glycol selectivity (%) | 1,2-Butylene glycol selectivity (%) |
|---|---|---|---|
| Ex. 10 | 41.8 | 16.9 | 21.3 |
| Ex. 11 | 37.7 | 15.5 | 21.6 |
| Ex. 12 | 40.9 | 16.2 | 21.5 |
| Ex. 13 | 35.2 | 11.5 | 14.3 |
| CE. 1 | 43.2 | 5.0 | 7.1 |
| CE. 2 | 42.0 | 6.5 | 8.2 |

ICP was used to quantitatively analyze the metal content in the reaction solutions obtained in Examples 10-18 and Comparative Examples 3-4. The results are shown in Table 4.

**Table 4**

| Catalyst | Glucose conversion rate (%) | Ni loss rate (%) | W loss rate (%) |
|---|---|---|---|
| Ex. 1 | >99 | 2 | 2.4 |
| Ex. 2 | >99 | 1.8 | 2.3 |
| Ex. 3 | >99 | 2.1 | 2.4 |
| Ex. 4 | >99 | 1.5 | 2.5 |
| Ex. 5 | >99 | 2.3 | 2.1 |
| Ex. 6 | >99 | 2.2 | 2.2 |
| Ex. 7 | >99 | 1.9 | 2.5 |
| Ex. 8 | >99 | 2.8 | 3.0 |
| Ex. 9 | >99 | 2.7 (Cu) | 3.3 (Mo) |
| CE. 1 | >99 | 12.5 | 31 |
| CE. 2 | >99 | 8.1 | 25 |

As shown in Table 4, the catalyst with dual catalytic centers according to the present invention has the advantages of low loss rate of active components and excellent cycling stability.

### Example 19

The catalyst prepared in Example 1 was washed and dried before being put into the next reaction. The reaction was repeated 4 times in total. The results are shown in Table 5. 0.075 g of the Ni_{15%}W_{15%}-C₃N₄/AC catalyst prepared in Example 1, 0.25 g of furfural, and 25 mL of deionized water were added to an autoclave with a stirrer, hydrogen was introduced to purge the autoclave three times, followed by pressurization with hydrogen to 4 MPa, after which the autoclave was sealed. The heating mantle was raised to a preset temperature, and then the stirrer was initiated by magnetic stirring. The reaction solution was subjected to gas phase analysis. The reaction was carried out at 245°C for 1 h. The products in the reaction solution were quantitatively analyzed by using gas chromatography, and the raw material in the reaction solution was quantitatively analyzed by using liquid chromatography.

**Table 5**

| Cycle times | Glucose conversion rate/% | Glycol yield/% |
|---|---|---|
| 1 | >99 | 80.0 |
| 2 | >99 | 75.1 |
| 3 | >99 | 77.8 |
| 4 | >99 | 77.3 |

As shown in Table 5, the catalyst with dual catalytic centers according to the present invention has high catalyst stability and no deactivation of the catalyst is observed after four cycles of use.

The preferred embodiments of the present invention are described in detail above, but the present invention is not limited thereto. Within the technical concept of the present invention, the technical solution of the present invention can be subjected to a variety of simple modifications, including the combinations of various technical features in any other suitable manner. These simple modifications and combinations should also be regarded as the contents disclosed by the present invention and fall within the protection scope of the present invention.

## Claims

1. A heterogeneous catalyst with dual catalytic centers, **characterized in that** the catalyst comprises:
a porous support and a heterogeneously distributed ligand, a heterogeneously distributed hydrogenation metal and a heterogeneously distributed retro-aldol catalytic metal loaded on the porous support; the hydrogenation metal and the retro-aldol catalytic metal form clusters with the ligand, wherein the hydrogenation metal and the retro-aldol catalytic metal coordinate with the coordination element of the ligand, and form aggregates having an average particle diameter in the range of 5-100 nm with the ligand.

2. The catalyst according to claim 1, wherein the ligand is C₃N₄ or a derivative thereof, wherein the C₃N₄ derivative comprises heteroatom doped carbon nitrides, defective carbon nitrides, carbon nitride heterojunctions or carbon nitride copolymers; preferably, the heteroatom is selected from phosphorus and sulfur.

3. The catalyst according to claim 1 or 2, wherein the clusters have an average particle diameter in the range of 5-30 nm.

4. The catalyst according to any one of the preceding claims, wherein the hydrogenation metal is loaded on the porous support in the form of active single-site of the metal, and preferably the retro-aldol catalytic metal is also loaded on the porous support in the form of active single-site of the metal.

5. The catalyst according to any one of the preceding claims, wherein the porous support has an average particle diameter in the range of 0.01-1 mm, preferably 0.1-0.5 mm; and/or
the porous support has a specific surface area in the range of 200-2000 m²•g⁻¹.

6. The catalyst according to any one of the preceding claims, wherein, based on the total weight of the catalyst, the content of the hydrogenation metal is in the range of 1%-50%, preferably 6%-30%; and/or
the content of the retro-aldol catalytic metal is in the range of 0.1%-50%, preferably 10%-30%; and/or
the content of the porous support is in the range of 25-96.9%, preferably 40-84%; and/or
the content of the ligand is in the range of 2-50%, preferably 10-30%; and/or
the weight ratio of the retro-aldol catalytic metal to the hydrogenation metal is in the range of 0.5-6, preferably 0.8-2.5.

7. The catalyst according to any one of the preceding claims, wherein the porous support is selected from carbon-based porous supports, preferably selected from at least one of graphene, graphyne, activated carbon fiber, activated carbon and carbon nanotubes; and/or
the hydrogenation metal is selected from at least one of Ni, Fe, Cu, Pt, Pd, Ru and Rh; and/or
the retro-aldol catalytic metal is selected from at least one of W, Mo and Ce.

8. A method for preparing the catalyst according to any one of the preceding claims, **characterized in that** the method comprises the following steps:
(1) dissolving a retro-aldol catalytic metal source, a hydrogenation metal source, and a ligand precursor in a solvent, followed by loading onto a porous support through contact with the porous support;
(2) calcining the product obtained in step (1) under an inert gas atmosphere.

9. The method according to claim 8, wherein the solvent is selected from at least one of water, methanol, ethanol and ethyl acetate, preferably water and/or methanol.

10. The method according to claim 8 or 9, wherein, step (1) comprises:
i) dissolving the retro-aldol catalytic metal source and the ligand precursor in a first solvent to obtain a first solution; dissolving the hydrogenation metal source and the ligand precursor in a second solvent to obtain a second solution;
ii) impregnating the porous support with the first solution and the second solution, respectively, followed by drying;
preferably,
the first solvent is methanol and the second solvent is water; and/or
the impregnation is carried out by equal volume impregnation(s); and/or
the calcination conditions include: a calcination temperature of 300-600°C and a calcination time of 0.5-12 hours.

11. The method according to any one of claims 8 to 10, wherein:
the weight ratio of the hydrogenation metal source to the ligand precursor is in the range of (0.5-10):1, preferably (1-3):1; and/or
the weight ratio of the retro-aldol catalytic metal source to the ligand precursor is in the range of (0.5-10):1, preferably (1-3):1.

12. The method according to any one of claims 8 to 11, wherein the product obtained in step (1) is pretreated before calcination in step (2), and the pretreatment conditions include: treating at 30-100°C for 0.25-24 hours under an inert gas or air atmosphere.

13. The method according to any one of claims 8 to 12, wherein:
the ligand precursor is selected from at least one of urea, dicyandiamide, melamine and guanidine hydrochloride; and/or
the hydrogenation metal source is selected from soluble salts of the hydrogenation metal, preferably at least one of hydrogenation metal acetates, hydrogenation metal nitrates, hydrogenation metal chlorides and hydrogenation metal sulfates, preferably at least one of nickel acetate, nickel nitrate, nickel chloride and nickel sulfate; and/or
the retro-aldol catalytic metal source is selected from at least one of soluble salts of the retro-aldol catalytic metal, soluble acids of the retro-aldol catalytic metal, and soluble oxides of the retro-aldol catalytic metal, preferably at least one of tungstic acid, ammonium metatungstate, and tungsten oxide; and
the hydrogenation metal is selected from at least one of Ni, Fe, Cu, Pt, Pd, Ru and Rh; and /or
the retro-aldol catalytic metal is selected from at least one of W, Mo and Ce.

14. Use of the catalyst according to any one of claims 1 to 7 in the production of glycols from a polyhydroxy compound.

15. A method for producing glycols, which comprises:
hydrolyzing and hydrogenating a polyhydroxy compound in the presence of hydrogen and the catalyst according to any one of claims 1 to 7 to produce glycols;
preferably,
the weight ratio of the polyhydroxy compound to the catalyst is in the range of 10-0.1; and/or
the weight ratio of water to the polyhydroxy compound is in the range of 30-300:1; and/or
the initial filling hydrogen pressure is in the range of 0.5-10 MPa; and/or
the hydrolysis and hydrogenation conditions include: a reaction temperature of 150-300°C; a reaction time of 0.5-12 h; and/or
the polyhydroxy compound is selected from at least one of starch, hemicellulose, sucrose, glucose, fructose, furfural and fructan.
